(19) Europäisches Patentamt
European Patent Office
Office européen des brevets

(11) Publication number: **0 349 487 A2**

# (12) EUROPEAN PATENT APPLICATION

(21) Application number: 89810477.3

(22) Date of filing: 20.06.89

(51) Int. Cl.⁵: **A 61 L 2/18**
A 01 N 33/12, A 61 K 9/08

(30) Priority: 28.06.88 US 212486

(43) Date of publication of application:
03.01.90 Bulletin 90/01

(84) Designated Contracting States:
AT BE CH DE ES FR GB GR IT LI LU NL SE

(71) Applicant: CIBA-GEIGY AG
Klybeckstrasse 141
CH-4002 Basel (CH)

(72) Inventor: Heyl, Barbara L.
516 Sidney Street
Atlanta Georgia 30312 (US)

The other inventors have agreed to waive their entitlement to designation

(54) **Antimicrobial ophthalmic solutions containing dodecyl-dimethyl-(2-phenoxyethyl)-ammonium bromide and methods of using the same.**

(57) The invention relates to the use of dodecyl-dimethyl-(2 phenoxyethyl)-ammonium bromide as an antimicrobial agent to be a preservative for ophthalmic drug solutions, in disinfecting solutions, as well as in preservatives for aqueous ocular solutions for contact lenses.

EP 0 349 487 A2

**Description**

## ANTIMICROBIAL OPHTHALMIC SOLUTIONS CONTAINING DODECYL-DIMETHYL-(2-PHENOXYETHYL)-AMMONIUM BROMIDE AND METHODS OF USING THE SAME

The present invention relates to ophthalmic solutions utilizing dodecyl-dimethyl-(2-phenoxyethyl)-ammonium bromide.

The eye, and particularly the conjunctiva, offers an ideal environment for many types of organisms. Infections of the eye can be very serious. For example, pseudomonas aeruginosa is a particularly dangerous and opportunistic organism, and has been known to cause infections which progress so rapidly that loss of the eye can occur in as short a period as twenty-four hours after onset. For this reason, it is important that solutions used in connection with the eye, such as preserved ophthalmic solutions and contact lens disinfecting solutions, be as effective antimicrobial agents as possible.

Presently, there is not a problem-free preservative available for ophthalmic use. Preservatives presently in use in ophthalmic solutions include benzalkonium chloride, chlorhexidine, thimerosal and chlorobutanol. However, at the concentrations needed to be effective, these compounds have high ocular toxicity or sensitivity. For example, if used repeatedly in concentrations of 1:5000 or stronger, benzalkonium chloride can denature the corneal protein causing irreversible damage. Other known preservatives, such as Onamer M and sorbic acid, are not very effective and therefore have short product shelf lives.

There exists, therefore, a need for improved ophthalmic solutions which are both effective as antimicrobial agents and which are also ocularly compatible.

There are several patents which disclose the use of quaternary ammonium compounds in contact lens solutions, including U.S. Patent Nos. 3,549,747 (Krezanoski et al.), 3,639,576 (Kaspar et al.), 4,013,576 (Loshaek), 4,029,817 (Blanco et al.), 4,039,662 (Hecht et al.) and 4,361,548 (Smith et al.), but all of the disclosed compositions have certain disadvantages.

There also exists a need for improved ophthalmic solutions which have a longer shelf life.

In accordance with this invention, there is provided an ophthalmic solution having as the active antimicrobial agent dodecyl-dimethyl-(2 phenoxyethyl)-ammonium bromide. The compound has been found to be effective, for example, as a preservative in saline at concentrations as low as 5 ppm, and as a disinfectant at concentrations as low as 30 ppm.

It has also been found that the presence of ethylenediaminetetraacetic acid, or EDTA, in addition to the dodecyl-dimethyl-(2 phenoxyethyl)-ammonium bromide, leads to increased antimicrobial action far exceeding that previously noted for quaternary ammonium compounds.

Typically, a solution utilizing the present invention may be a preserving and/or disinfecting solution for contact lenses, or an ophthalmic drug solution. Such solutions prepared according to the present invention have been found to be both effective as antimicrobial agents, as well as ocularly compatible.

Dodecyl-dimethyl-(2 phenoxyethyl)-ammonium bromide which is also known as domiphen bromide has the formula:

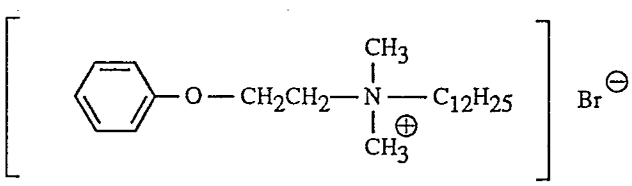

and is available commercially e.g. as Bradosol®, a trade mark of Ciba-Geigy Corporation. Bradosol® belongs to a family of compounds known as quaternary ammonium salts, and has high bactericidal, bacteriostatic and fungicidal activity, even at very low concentrations. It has been used previously in medical applications including surgical scrubs, instrument storage and throat lozenges.

It has now been found that dodecyl-dimethyl-(2 phenoxyethyl)-ammonium bromide is significantly less irritating than other antimicrobial agents, and particularly other quaternary compounds used in ophthalmic solutions. It has also been found that, in combination with ethylenediaminetetraacetic acid, or EDTA, its antimicrobial properties are greatly en hanced. As such, dodecyl-dimethyl-(2 phenoxyethyl)-ammonium bromide can be effectively used as a preservative for ophthalmic drug solutions, or in disinfecting solutions, as well as a preservative for aqueous ocular solutions for contact lenses.

It has also been found that solution component interaction is important to the effectiveness of dodecyl-dimethyl-(2 phenoxyethyl)-ammonium bromide in solutions such as suggested here. For example, Bradosol® is compatible with lactate, tartrate, phosphate, borate, carbonate, and citrate buffer systems to provide the proper pH for use in the eye. The solution is typically maintained at an osmotic pressure similar to that of physiological saline (0.9 % saline). However, the presence of polymers such as polyvinyl alcohol causes a decrease in effectiveness, so higher concentrations of the Bradosol® are needed to preserve such a solution. The same is true for surfactants such as those used in contact lens cleaning solutions. It is believed that this is a result of the interaction of the quaternary nitrogen (positively charged) with various groups,

thereby leaving less dodecyl-dimethyl-(2 phenoxyethyl)-ammonium bromide free in solution. Because the nitrogen is positively charged, components with a strong negative charge bind the Bradosol® completely. As such, negatively charged compounds or drugs are not effectively preserved according to the present invention.

The invention is directed to an ophthalmic solution comprising an effective amount of dodecyl-dimethyl-(2 phenoxyethyl)-ammonium bromide as an antimicrobial agent. Preferably, in such a solution, said dodecyl-dimethyl-(2 phenoxyethyl)-ammonium bromide is present in an amount of approximately 0.0005 % to 1.0 weight percent of said solution.

Preferably said ophthalmic solution further comprises EDTA. When present, said EDTA is present in an amount of approximately 0.1 weight percent of said solution.

Preferably the ophthalmic solution according to the invention further comprises an effective amount of a buffer to provide said solution with an ophthalmically acceptable pH.

Further preferred is an ophthalmic solution comprising dodecyl-dimethyl-(2 phenoxyethyl)-ammonium bromide in an amount of at least 0.0005 weight percent of said solution, wherein said solution is a preserved saline solution.

Even said preserved saline solution as defined hereinbefore as being further preferred, preferably further comprises EDTA, advantageously in an amount of approximately 0.1 weight percent of said solution.

The invention is even directed to a method of imparting antimicrobial activity to an ophthalmic solution, comprising adding to said solution an effective amount of dodecyl-dimethyl-(2 phenoxyethyl)-ammonium bromide as an antimicrobial agent. In said method, any of the ophthalmic solutions, as mentioned hereinbefore, may be utilized. Preferably, the lower limit of dodecyl-dimethyl-(2 phenoxyethyl)-ammonium bromide is raised to 0.001 weight percent of said solution.

Further, in a method of preserving a saline solution, it is advantageous to comprise the step of adding to said solution dodecyl-dimethyl-(2 phenoxyethyl)-ammonium bromide in an amount of at least approximately 0.0040 weight percent of said solution.

However, in said method of preserving a saline solution, it is even within the scope of the disclosure to comprise the steps of adding to said solution dodecyl-dimethyl-(2 phenoxyethyl)-ammonium bromide in an amount of at least approximately 0.0005 weight percent of said solution and further adding EDTA in an amount of approximately 0.1 weight percent of said solution.

The EDTA level can be modified up to a maximum of 0.2 % (by weight) and as low as 0%.

It was found that EDTA, in combination with Bradosol®, and particularly at approximately 0.1 % (wt/vol.) has a synergistic effect and gives a greater increase in effectiveness to the Bradosol® than previously noted for quaternary ammonium compounds.

In the presence of EDTA, dodecyl-dimethyl-(2 phenoxyethyl)-ammonium bromide has been found to be effective as a preservative in saline as low as 10 ppm. When there is interaction of the Bradosol® with other components of a solution, as in the examples provided, a higher concentration is preferred.

It has also been determined that dodecyl-dimethyl-(2 phenoxyethyl)-ammonium bromide has relatively low ocular toxicity. Rabbit testing was performed using standard Draize testing. Using benzalkonium chloride at 40 ppm as a control, Bradosol® was administered to eyes at concentrations of 100, 500 and 1000 ppm. No reaction was seen at 1000 ppm. Benzalkonium chloride, when administered ocularly at 1000 ppm, caused severe reaction and could only be administered once. Using Bradosol®, no reaction was seen after administration over 21 days at 100 ppm.

The invention is further directed to an ophthalmic solution, wherein said solution is an ophthalmic drug solution, and wherein said dodecyl-dimethyl-(2 phenoxyethyl)-ammonium bromide is present in an amount from approximately 0.001 % to 1.0 %.

Examples of ophthalmic drugs which can use Bradosol® as a preservative, typically at a range between 0.001 % and 1 %, include:

| DRUG | USE |
|---|---|
| Proparacaine·HCl | Anesthetic |
| Tetracaine·HCl | Anesthetic |
| Carbachol | Glaucoma |
| Pilocarpine | Glaucoma |
| Physostigmine·$SO_4$ | Glaucoma |
| Homatropine | Mydriatic |
| Idoxuridine | Herpes |
| Dexamethasone | Inflammation |
| Prednisolone | Inflammation |
| Epinephrine·HCl | Glaucoma |
| Epinephrine·borate | Glaucoma |
| Phenylephrine·HCl | Decongestant |
| Atropine sulfate | Mydriasis |
| Cyclopentolate·HCl | Mydriasis |

The invention is further directed to an ophthalmic solution, wherein said solution is a cleaner for contact lenses, and wherein said dodecyl-dimethyl-(2 phenoxyethyl)-ammonium bromide is present in an amount of at least approximately 0.0080 weight percent of said solution.

A preferred preserved cleaner for contact lenses comprises:

a) from approximately 0.0080 to 1.0 weight percent of dodecyl-dimethyl-(2 phenoxyethyl)-ammonium bromide;

b) an effective amount of buffer to provide said solution with an ophthalmically acceptable pH;

c) an effective amount of an organic or inorganic salt, preferably NaCl, to provide isotonicity to said solution;

d) an effective amount of a surfactant; and

e) an effective amount of EDTA.

A suitable buffer system is e.g. a phosphate buffer, although a borate, citrate, acetate or Tris buffer system, or combinations thereof, can be used with equal efficacy. The concentration of the buffer can be easily modified and specified by those familiar with buffering systems. The system should be such as to keep the Bradosol® within a range of 6.5-8.0, with the preferred range being near to pH 7.0-7.4, where it is most effective.

Preferably sodium chloride is used to adjust the osmolarity of the solution to a desired level, advantageously to isotonicity. Other inorganic or organic salts may be used, but sodium chloride is preferred for ease of obtaining a pure, reproducible salt. The osmolarity of such a solution, e.g. that of Example 1, can be adjusted to $300 \pm 50$ without any apparent disadvantage.

A surfactant useful in a preserved cleaner according to the invention is e.g. any "true" non-ionic surfactant, e.g. amine oxide surfactants that exhibit little or no affinity to the dodecyl-dimethyl-(2 phenoxyethyl)-ammonium bromide. The amount of an amine oxide surfactant used may range from 0.3 % to 3.0 % while approximately 1.0 % as set forth e.g. in example 1, is considered optimal. A preferred amine oxide surfactant is Varox® 365, a trademark of Sherex Chemical, used for solubilizing grease and dirt that typically do not naturally occur in vivo, such as cosmetics and body oils. Furthermore, Varox® 365 is a good foaming agent, which is desirable in a cleaning solution.

Furthermore, a surfactant such as Triton® X-100, a trademark of Rohm and Haas, which is primarily used as a surfactant for proteins, lipids, dirt, and other debris that occur in vivo with contact lens wear is useful in the instant solutions. Most of the Triton series could be used, yet Triton® X-100 is preferred. Although it is widely held that Triton® is potentially dangerous for use in the eye, the percentage used e.g. in the solution of Example 1 has shown no more than transient reddening of the eye that clears within one hour. It has been found that an increase in the amount of detergent in the solution requires an increase in the amount of Bradosol® used.

The EDTA level can be modified up to a maximum of 0.2 % (by weight) and as low as 0%.

EDTA is used for two purposes. First, it acts as a chelating agent for ions that typically adhere to contact lenses and form undesired deposits. Second, it was found that EDTA, in combination with Bradosol®, and particularly at approximately 0.1 % (wt/vol.) has a synergistic effect and gives a greater increase in effectiveness to the Bradosol® than previously noted for quaternary ammonium compounds.

The preserved cleaner as defined hereinbefore may comprise, additionally, a viscosity builder. As a viscosity builder, any of the cellulosic derivatives such as Cellosize® QP-40, a trademark of Union Carbide, being a quick dissolving hydroxyethyl cellulose could be used to adjust the viscosity of the solution to a desired level, as may be other viscosity builders such as the Carbopols, to provide the preferred viscosity of from 3 to 25 cps (Brookfield Spindle).

Preferred is a preserved cleaner, wherein said solution comprises approximately 0.0080 to 1.0 weight percent of dodecyl-dimethyl-(2 phenoxyethyl)-ammonium bromide, approximately 1.0 weight percent of a phosphate buffer, approximately 0.1 weight percent EDTA, approximately 0.35 weight percent NaCl, from

approximately 0.3 to 3.0 weight percent surfactant, and a viscosity builder.

The invention is further directed to an ophthalmic solution, wherein said solution is a conditioner for contact lenses and wherein said dodecyl-dimethyl-(2 phenoxyethyl)-ammonium bromide is present in an amount from approximately 0.0035 to approximately 1.0 weight percent of said solution. Such a solution has said dodecyl-dimethyl-(2 phenoxyethyl)-ammonium bromide e.g. present in an amount of approximately 0.0050 weight percent of said solution. Said solution, being a conditioner for contact lenses may have EDTA present in an amount of e.g. approximately 0.1 weight percent of said solution.

A further embodiment of the invention is a conditioning solution for contact lenses comprising:

a) an effective amount of dodecyl-dimethyl-(2 phenoxyethyl)-ammonium bromide;

b) an effective ammount of a buffer to provide said solution with an ophthalmically acceptable pH;

c) an effective amount of an organic or inorganic salt, preferably NaCl to provide isotonicity to said solution; and

d) an effective amount of a wetting agent.

In said conditioning solution said dodecyl-dimethyl-(2 phenoxyethyl)-ammonium bromide is present preferably in an amount of from approximately 0.0035 to 1.0 weight percent.

Advantageously the solution further contains EDTA. More preferred, said EDTA is present in an amount of approximately 0.1 weight percent of said solution, and the solution contains approximately 0.0050 % (wt/vol.) of Bradosol®. The wetting exhibited by such a solution, a specific example is presented in example 2, is superior to all known conditioners, and is retained for longer than would be expected from such a solution.

The EDTA and sodium chloride are used in a similar fashion as set forth in detail in the description of cleaner solutions for contact lenses.

Suitable wetting agents are polyvinyl alcohols (PVA) or combinations thereof with polyvinyl pyrrolidone (PVP).

A preferred wetting agent is Vinol® 350 PVA which is a trademark of Air Products, Inc. It is preferred in the disinfecting and soaking solution to use specified grades of PVA. PVA exhibits better wetting as its molecular weight increases and as its degree of hydrolysis decreases. However, these characteristics present manufacturing problems in that increased molecular weight results in slower solubilization and more viscous solutions, while as the degree of hydrolysis decreases, the solubilization of PVA decreases. It has been found that, for best results in a solution such as e.g. Example 2, a 70 % hydrolyzed product should be used. The concentration of PVA is effective at amounts as high as 3 % by weight and as low as 0.25 % by weight. As the concentration of PVA drops below 1.0 % by weight, the concentration of e.g. Plasdone K-90 PVP must be increased to offset the losses of wettability.

Plasdone K-90 PVP, a product of GAF, or povidone, has been found to have a synergistic wetting effect when used in combination with Vinol® 350 PVA. While Plasdone K-30 may be used, it is not as effective as the K-90. The concentration of PVP depends upon the concentration of PVA utilized as noted above. At amounts above 0.75 % by weight PVA, there is no discernable advantage by using more than equivalent percentages of PVP, although at this level one should not use less than the PVA concentration. Below 0.75 % by weight, various properties were shown with differing PVA/PVP concentrations. PVP and PVA have been used in concentrations of from 0.25 % to 3.0 % by weight.

The phosphate buffering system is important to a solution such as that of Example 2, since the PVA is incompatible with borate, citrate, and acetate buffering systems. However, any PVA compatible buffer may be used.

It must be noted that a disinfecting and soaking solution such as set forth e.g. in Example 2 is not recommended for use with HEMA lenses. The Bradosol® is present at such concentrations as to be taken up and concentrated into the matrix of these lenses upon extended contact time; i.e., greater than 30 minutes. Such concentrations of Bradosol® may result in cytotoxicity.

A further subembodiment of the present invention is a conditioning solution for contact lenses as described hereinbefore, wherein said dodecyl-dimethyl-(2 phenoxyethyl)-ammonium bromide is present in an amount of from at least approximately 0.0015 to approximately 1.0 weight percent and further comprising an effective amount of catalase. Such a solution is referred to herein as a neutralizing conditioner.

A preferred neutralizing conditioner has catalase present in an amount of at least approximately 0.03 weight percent of said solution.

It has been found that the catalase enhances the effectiveness of the Bradosol®. In preparation, the buffer, e.g., $Na_2HPO_4$, $NaH_2PO_4 \cdot H_2O$, $Na_2(EDTA) \cdot 2H_2O$, Bradosol®, and NaCl should be added separately and stirred until dissolution is complete. The temperature of the broth should remain at ambient during the process. The wetting agent, e.g. Vinol® 165 (or Vinol® 350) is then added and the solution is stirred vigorously. The Plasdone K-90 if used, should be added once the Vinol® has formed a suspension with the solution. After the addition of the Plasdone K-90, the solution should be heated to 85-86° C. Dissolution of these items will take from about 30 to 50 minutes. For sterilization, the solution can be autoclaved at 121° C for 30 minutes. The catalase should be micro filtered (0.2 µm) into the main reactor only after the solution has reached 20-21° C.

The invention is further directed to an ophthalmic solution, wherein said solution is a disinfecting solution and wherein said dodecyl-dimethyl-(2 phenoxyethyl)-ammonium bromide is present in an amount of at least 0.0030 weight percent of said solution. The composition of such disinfecting solution is presented hereinafter referring to a method of disinfecting a contact lens with said solution.

The invention further comprises a method of disinfecting a contact lens, comprising the step of adding said

lens to a solution comprising an effective amount of dodecyl-dimethyl-(2 phenoxyethyl)-ammonium bromide. Advantageously in this method said dodecyl-dimethyl-(2 phenoxyethyl)-ammonium bromide is present in said solution in an amount from approximately 0.0035 to approximately 1.0 weight percent of said solution, an amount of approximately 0.0050 weight percent of said solution being preferred. Again, it is preferred that said solution further comprises EDTA present in an amount of approximately 0.1 weight percent of said solution.

It has been found that only a slightly higher concentration of Bradosol® is required for disinfection than for a preservative; the distinction being the ability not just to prevent growth of organisms in the solution, but to kill ones actually introduced. It was found that a conditioner formula could obtain rapid kill times (especially with bacteria) using concentrations of 50 ppm Bradosol®. This is important since most conditioning type solutions are not disinfecting as well.

In saline, effective disinfection was achieved at concentrations as low as 30 ppm.

While the above description contains many specificities, these should not be construed as limitations on the scope of the invention, but rather as exemplifications of certain preferred embodiments thereof. It will be understood that variations and modifications can be effected within the spirit and scope of the invention as previously described and as defined by the claims.

The following examples of ophthalmic solutions using dodecyl-dimethyl-(2 phenoxyethyl)-ammonium bromide are illustrative only and should not be construed as limiting the invention. Amounts are presented in weight percent if not stated otherwise.

EXAMPLE 1: Preserved Cleaner For Contact Lenses

| | |
|---|---|
| $Na_2HPO_4$ | 0.7287 % (wt/vol) |
| $NaH_2PO_4 \cdot H_2O$ | 0.2116 % (wt/vol) |
| $Na_2(EDTA) \cdot 2H_2O$ | 0.1000 % (wt/vol) |
| Bradosol® [dodecyl-dimethyl-(2 phenoxyethyl)-ammonium bromide] | 0.0080 % (wt/vol) |
| NaCl | 0.3552 % (wt/vol) |
| Varox® 365 | 1.0000 % (vol/vol) |
| Triton® X-100 | 0.3333 % (vol/vol) |
| Cellosize® QP-40 HEC | 1.5000 % (wt/vol) |
| Purified $H_2O$ | q.s. 100 ml |

The preserved cleaner set forth in this example, which utilizes a preferred minimum of approximately 80 ppm Bradosol®, has a pH of $7.20 \pm 0.1$ and has an osmolarity of $300 \pm$ 10 mOsm/kg. Although the formulation contains Bradosol®, it is found to be effective and compatible with all types of contact lenses, including those containing HEMA. It is believed that the short contact time for cleaning and the disinfection step which follows for HEMA lenses results in less absorption into the lens.

The following Table I summarizes preservative effectiveness tests utilizing the cleaner of this example (CFU is: Colony forming unit):

Table I

Number of Surviving Microorganisms (CFU ml)

| Microorganism | Initial (0) | 6h | 24h | 48h | 7d | 14d | 21d | 28d |
|---|---|---|---|---|---|---|---|---|
| Aspergillus niger | $10^6$ | - | - | - | $10^3$ | $10^2$ | $10^2$ | $10^2$ |
| Candida albicans | $10^6$ | - | - | - | 0 | 0 | 0 | 0 |
| Pseudomonas aeruginosa | $10^6$ | 0 | 0 | 0 | 0 | 0 | 0 | 0 |
| Staphylococcus aureus | $10^6$ | 0 | 0 | 0 | 0 | 0 | 0 | 0 |

EXAMPLE 2: Conditioning Solution for Gas Permeable and Hard Contact Lenses

| | |
|---|---|
| $Na_2HPO_4$ | 0.7193 % (wt/vol) |
| $NaH_2PO_4 \cdot H_2O$ | 0.2208 % (wt/vol) |
| $Na_2(EDTA) \cdot 2H_2O$ | 0.1 % (wt/vol) |
| Bradosol® [dodecyl-dimethyl-(2 phenoxyethyl)-ammonium bromide] | preferably 0.0050 % (wt/vol) |
| | (a range of 0.0035 % - 1.0 %) |
| NaCl | 0.4383 % (wt/vol) |
| Vinol® 350 PVA | 0.5 % (wt/vol) |
| Plasdone K-90 PVP | 1.0 % (wt/vol) |
| Purified $H_2O$ | q.s. 100 ml |

The solution for disinfecting and soaking gas permeable contact lenses set forth in this example has a pH of 7.40±0.1 and an osmolarity of 300±100 mOsm/kg. The particular solution used in Example 2 contains approximately 0.0035 % to 1.0 % (wt/vol) of Bradosol®, and preferably 0.0050% (wt/vol). The wetting exhibited by this solution is superior to all known conditioners, and is retained for longer than would be expected from such a solution.

The following Table II presents a summary of preservative effectiveness tests utilizing the formulation of this example:

Table II

Number of Surviving Microorganisms (CFU ml)

| Microorganism | Initial (0) | 6h | 24h | 48h | 7d | 14d | 21d | 28d |
|---|---|---|---|---|---|---|---|---|
| Aspergillus niger | $10^6$ | - | - | - | $10^4$ | $10^2$ | $10^2$ | $10^2$ |
| Candida albicans | $10^6$ | - | - | - | 0 | $10^2$ | 0 | 0 |
| Pseudomonas aeruginosa | $10^6$ | 0 | 0 | 0 | 0 | $10^2$ | 0 | 0 |
| Staphylococcus aureus | $10^6$ | 0 | 0 | 0 | 0 | 0 | 0 | 0 |

This solution is not recommended for use with HEMA lenses.

EXAMPLE 3: Neutralizing Conditioner for Hard Contact Lenses

| | |
|---|---|
| $Na_2HPO_4$ | 0.7193 % (wt/vol) |
| $NaH_2PO_4 \cdot H_2O$ | 0.2208 % (wt/vol) |
| $Na_2(EDTA) \cdot 2H_2O$ | 0.1 % (wt/vol) |
| Bradosol® [dodecyl-dimethyl-(2 phenoxyethyl)-ammonium bromide] | 0.0015 % (wt/vol) |
| NaCl | 0.3214 % (wt/vol) |
| Vinol® 165 (or Vinol® 350) | 1.0 % (wt/vol) |
| Plasdone K-90 PVP | 1.0 % (wt/vol) |
| Catalase | 700 units/ml |
| $H_2O$ | q.s. 100 ml |

The conditioner set forth in this Example has a pH of 7.20±0.1 and has an osmolarity of 300±10 mOsm/kg. The amount of Bradosol® used ranges from approximately 0.0015 % (wt/vol) to approximately 1 % (wt/vol).

The following Table III presents a summary of preservative effectiveness tests utilizing the formulation of this Example:

Table III

Number of Surviving Microorganisms (CFU ml)

| Microorganism | Initial (0) | 6h | 24h | 48h | 7d | 14d | 28d |
|---|---|---|---|---|---|---|---|
| Aspergillus niger | $6.5x10^4$ | - | - | - | $3.9x10^4$ | $2.9x10^2$ | $2.4x10^2$ |
| Candida albicans | $8.3x10^5$ | - | - | - | 0 | 0 | 0 |
| Pseudomonas aeruginosa | $2.7x10^6$ | 0 | 0 | 0 | 0 | 0 | 0 |
| Staphylococcus aureus | $3.0x10^6$ | 0 | 0 | 0 | 0 | 0 | 0 |

EXAMPLE 4: Summary of Ranges of Effectiveness

In the presence of EDTA, dodecyl-dimethyl-(2 phenoxyethyl)-ammonium bromide has been found to be effective as a preservative in saline as low as 10 ppm. When there is interaction of the Bradosol® with other components of a solution, as in the examples provided, a higher concentration is preferred. In the cleaner of Example 1, the level was found to be 80 ppm. In the conditioner of Example 3, the level is 15 ppm. When relatively small changes in the formulation are made, the Bradosol® must be adjusted to ensure a preserved solution. Examples of solutions which have been tested and results of BP testing are shown in Table IV:

TABLE IV

| Solution | Bradosol® | Pass/Fail |
|---|---|---|
| saline + EDTA | 10 ppm | P |
| saline + EDTA | 20 ppm | P |
| saline + EDTA | 50 ppm | P |
| saline + EDTA | 75 ppm | P |
| saline | 40 ppm | P |
| conditioner (PVP + PVP) + catalase | 15 ppm | P |
| conditioner + catalase | 30 ppm | P |
| conditioner/no catalase | 15 ppm | F |
| conditioner/no catalase | 30 ppm | P |
| conditioner/no catalase | 50 ppm | P |
| cleaner (anionic detergent) | 100 ppm | F |
| cleaner (all nonionic) | 80 ppm | P |

Note: Table IV is based upon the following: British Pharmacopeia 1980 Edition Test Standards. A "pass" requires that there be at least a 1000 fold reduction after 6 hours.

Used organisms: Aspergillus niger, Candida albicans, Pseudomonas aeruginosa, Staphylococcus aureus; each solution must pass with respect to all in order to be given a "P" rating

Requirements state:
A three log reduction or greater in bacteria per ml within 6 hours, no bacteria at 24 hours or after. Fungi reduced by 2 logs within 7 days, no increase after).

EXAMPLE 5:
It was found that a conditioner formula could obtain rapid kill times (especially with bacteria) using concentrations of 50 ppm Bradosol®, as summarized in Table V, below.

TABLE V

Conditioner With Bradosol® Kill Times

| | Time (minutes) | | | | | |
|---|---|---|---|---|---|---|
| S.aureus Bradosol® | 0 | 1 | 3 | 5 | 15 | |
| .0015 % | $10^6$ | $10^6$ | $10^4$ | $10^4$ | $10^2$ | |
| .003 % | $10^6$ | $10^6$ | $10^4$ | $10^2$ | $10^1$ | |
| .005 % | $10^6$ | $10^5$ | $10^3$ | $10^2$ | $10^1$ | |
| .01 % | $10^6$ | $10^2$ | $10^1$ | $10^1$ | $10^1$ | |
| A.niger Bradosol® | 0 | | | | | 180 |
| .0015 % | $10^6$ | | | | | $10^5$ |
| .003 % | $10^6$ | | | | | $10^4$ |
| .005 % | $10^6$ | | | | | $10^2$ |
| .01 % | $10^6$ | | | | | $10^2$ |

In saline, disinfection was achieved at concentrations as low as 30 ppm, as summarized in Table VI below.

TABLE VI

Kill Times For Bradosol® Containing Saline

| Time (min.) | .003 % | .005 % | .007 % |
|---|---|---|---|
| | | P.aeruginosa | |
| 0 | $10^6$ | $10^6$ | $10^6$ |
| 5 | 0 | 0 | 0 |
| 10 | 0 | 0 | 0 |
| 15 | 0 | 0 | 0 |
| | | S.aureus | |
| 0 | $10^6$ | $10^6$ | $10^6$ |
| 5 | $10^3$ | $10^2$ | 0 |
| 10 | $10^1$ | 0 | 0 |
| 30 | 0 | 0 | 0 |

**Claims**

1. An ophthalmic solution comprising an effective amount of dodecyl-dimethyl-(2 phenoxyethyl)-ammonium bromide as an antimicrobial agent.
2. The ophthalmic solution of claim 1, wherein said dodecyl-dimethyl-(2 phenoxyethyl)-ammonium bromide is present in an amount from approximately 0.0005 % to 1.0 weight percent of said solution.
3. The ophthalmic solution of claim 1, and further comprising EDTA.
4. The ophthalmic solution of claim 3, wherein said EDTA is present in an amount of approximately 0.1 weight percent of said solution.
5. The ophthalmic solution of claim 1, wherein said solution further comprises an effective amount of buffer to provide said solution with an ophthalmically acceptable pH.
6. The ophthalmic solution of claim 1 , wherein said solution is a preserved saline solution and wherein said dodecyl-dimethyl-(2 phenoxyethyl)-ammonium bromide is present in an amount of at least 0.0005 weight percent of said solution.
7. The ophthalmic solution of claim 3, wherein said solution is a preserved saline solution and wherein said dodecyl-dimethyl-(2 phenoxyethyl)-ammonium bromide is present in an amount of at least approximately 0.0005 weight percent of said solution.
8. The ophthalmic solution of claim 7, wherein said EDTA is present in an amount of approximately 0.1 weight percent of said solution.
9. The ophthalmic solution of claim 1, wherein said solution is anophthalmic drug solution, and wherein said dodecyl-dimethyl-(2 phenoxyethyl)-ammonium bromide is present in an amount from approximately 0.001 % to 1.0 %.
10. The ophthalmic solution of claim1 , wherein said solution is a cleaner for contact lenses, and wherein said dodecyl-dimethyl-(2 phenoxyethyl)-ammonium bromide is present in an amount of at least approximately 0.0080 weight percent of said solution.
11. A preserved cleaner for contact lenses, comprising:
    a) from approximately 0.0080 to 1.0 weight percent of dodecyl-dimethyl-(2 phenoxyethyl)-ammonium bromide;
    b) an effective amount of buffer to provide said solution with an ophthalmically acceptable pH;
    c) an effective amount of an organic or inorganic salt, preferably NaCl, to provide isotonicity to said solution;
    d) an effective amount of a surfactant; and
    e) an effective amount of EDTA.
12. The preserved cleaner of claim 11, wherein said solution is comprised of approximately 1.0 weight percent of a phosphate buffer, approximately 0.1 weight percent EDTA, approximately 0.35 weight percent NaCl, from approximately 0.3 to 3.0 weight percent surfactant, and a viscosity builder.
13. The preserved cleaner of claim 11, wherein said surfactant is an amine oxide surfactant.
14. The ophthalmic solution of claim 1, wherein said solution is a conditioner for contact lenses and wherein said dodecyl-dimethyl-(2 phenoxyethyl)-ammonium bromide is present in an amount from approximately 0.0035 to approximately 1.0 weight percent of said solution.
15. The ophthalmic solution of claim 14, wherein said dodecyl-dimethyl-(2 phenoxyethyl)-ammonium bromide is present in an amount of approximately 0.0050 weight percent of said solution.

16. The ophthalmic solution of claim 14, and further comprising EDTA present in an amount of approximately 0.1 weight percent of said solution.

17. A conditioning solution for contact lenses comprising:

a) an effective amount of dodecyl-dimethyl-(2 phenoxyethyl)-ammonium bromide;

b) an effective ammount of a buffer to provide said solution with an ophthalmically acceptable pH;

c) an effective amount of an organic or inorganic salt, preferably NaCl to provide isotonicity to said solution; and

d) an effective amount of a wetting agent.

18. The conditioning solution of claim 17, wherein said wetting agent is polyvinyl alcohol.

19. The conditioning solution of claim 18, and further comprising povidone.

20. The conditioning solution of claim 18, wherein said polyvinyl alcohol is present at approximately 0.25 weight percent to approximately 3.0 weight percent.

21. The conditioning solution of claim 19, wherein the total amount of said polyvinyl alcohol and said povidone is approximately 0.25 weight percent to approximately 3.0 weight percent.

22. The conditioning solution of claim 17, wherein said dodecyl-dimethyl-(2 phenoxyethyl)-ammonium bromide is present in an amount of from approximately 0.0035 to 1.0 weight percent, and the solution further contains EDTA.

23. The conditioning solution of claim 22, wherein said EDTA is present in an amount of approximately 0.1 weight percent of said solution.

24. The ophthalmic solution of claim 1, wherein said solution is a neutralizing conditioner for contact lenses and said dodecyl-dimethyl-(2 phenoxyethyl)-ammonium bromide is present in an amount from approximately 0.0015 to approximately 1.0 weight percent of said solution, and wherein said solution further comprises an effective amount of catalase.

25. The ophthalmic solution of claim 24, wherein said catalase is present in an amount of at least approximately 0.03 weight percent of said solution.

26. The conditioning solution of claim 17, wherein said dodecyl-dimethyl-(2 phenoxyethyl)-ammonium bromide is present in an amount of from at least approximately 0.0015 weight per cent, and further comprising catalase.

27. The ophthalmic solution of claim 1, wherein said solution is a disinfecting solution and wherein said dodecyl-dimethyl-(2 phenoxyethyl)-ammonium bromide is present in an amount of at least 0.0030 weight percent of said solution.

28. A method of imparting antimicrobial activity to an ophthalmic solution, comprising adding to said solution an effective amount of dodecyl-dimethyl-(2 phenoxyethyl)-ammonium bromide as an antimicrobial agent.

29. The method of claim 28, wherein said dodecyl-dimethyl-(2 phenoxyethyl)-ammonium bromide is added in an amount of from approximately 0.001 to 1.0 weight percent of said solution.

30. The method of claim 28, and further comprising the step of adding an effective amount of EDTA.

31. The method of claim 30, wherein said EDTA is added in an amount of approximately 0.1 weight percent of said solution.

32. The method of claim 28, and further comprising the step of adding an effective amount of buffer to provide said solution with an ophthalmically acceptable pH.

33. A method of preserving a saline solution, comprising the step of adding to said solution dodecyl-dimethyl-(2 phenoxyethyl)-ammonium bromide in an amount of at least approximately 0.0040 weight percent of said solution.

34. A method of preserving a saline solution, comprising the steps of adding to said solution dodecyl-dimethyl-(2 phenoxyethyl)-ammonium bromide in an amount of at least approximately 0.0005 weight percent of said solution and further adding EDTA in an amount of approximately 0.1 weight percent of said solution.

35. The method of claim 28, wherein said solution is an ophthalmic drug solution, and wherein said dodecyl-dimethyl-(2 phenoxyethyl)-ammonium bromide is present in an amount from approximately 0.001% to 1.0%.

36. A method of cleaning a contact lens, comprising the step of contacting said lens with a solution wherein said dodecyl-dimethyl-(2 phenoxyethyl)-ammonium bromide is present in an amount of at least approximately 0.0080 weight percent of said solution.

37. A method of storing or conditioning a contact lens, comprising the step of adding said lens to a solution wherein said dodecyl-dimethyl-(2 phenoxyethyl)-ammonium bromide is present in an amount from approximately 0.0035 to approximately 1.0 weight percent of said solution.

38. A method of disinfecting a contact lens, comprising the step of adding said lens to a solution comprising an effective amount of dodecyl-dimethyl-(2 phenoxyethyl)-ammonium bromide.

39. The method of claim 38, wherein said dodecyl-dimethyl-(2 phenoxyethyl)-ammonium bromide is present in said solution in an amount from approximately 0.0035 to approximately 1.0 weight percent of said solution.

40. The method of claim 38, wherein said dodecyl-dimethyl-(2 phenoxyethyl)-ammonium bromide is present in said solution in an amount of approximately 0.0050 weight percent of said solution.

41. The method of claim 38, wherein said solution further comprises EDTA present in an amount of

approximately 0.1 weight percent of said solution.

42. The method of claim 38, wherein said dodecyl-dimethyl-(2 phenoxyethyl)-ammonium bromide is present in said solution in an amount from approximately 0.0015 to approximately 1.0 weight percent of said solution, and wherein said solution further comprises an effective amount of catalase.

43. The method of claim 42, wherein said catalase is present in said solution in an amount of at least approximately 0.03 weight percent of said solution.

44. Use of dodecyl-dimethyl-(2 phenoxyethyl)-ammonium bromide for imparting antimicrobial activity to an ophthalmic solution.

45. Use of dodecyl-dimethyl-(2 phenoxyethyl)-ammonium bromide for imparting antimicrobial activity to an ophthalmic drug solution.

46. Use of dodecyl-dimethyl-(2 phenoxyethyl)-ammonium bromide for imparting antimicrobial activity to a cleaner for contact lenses, to a conditioner for contact lenses or to a disinfecting solution, e.g. for contact lenses.

47. Use of dodecyl-dimethyl-(2 phenoxyethyl)-ammonium bromide for making an ophthalmic solution as disclosed in any of claims 1 to 27.

48. A process for manufacture of an ophthalmic solution as disclosed in any of Claims 1 to 27 characterized by conventional mixing of the ingredients.